# EUROPEAN PATENT APPLICATION

(11) **EP 3 546 060 A1**
(43) Date of publication of application: **02.10.2019**
(21) Application number: 18164250.5
(22) Date of filing: 27.03.2018
(51) Int. Cl.: B01J 2/02, C07H 1/06, C07H 3/06, A23L 33/00, A23L 33/21

(54) **PROCESS FOR SPRAY DRYING FUCOSYLLACTOSE SOLUTIONS AND RELATED PRODUCT COMPOSITIONS**

(71) Applicant: DuPont Nutrition Biosciences ApS, 1411 Copenhagen K (DK)
(72) Inventor: DALGAARD, Niels, 1411 Copenhagen K (DK)
(74) Representative: DuPont EMEA

(57) **Abstract**

This specification relates to a process for preparing a dried fucosyllactose (FL) from an FL solution by spray drying. This specification also relates to an FL in powder form having high bulk density, such as is obtained by the process disclosed in this specification, as well as prebiotics, probiotics and foods comprising the FL.

## Description

### FIELD

This specification relates to a process for preparing a dried fucosyllactose (FL) from an FL solution by spray drying. This specification also relates to an FL in powder form having high bulk density, such as is obtained by the process disclosed in this specification, and to prebiotics, probiotics and foods comprising the FL.

### BACKGROUND

Sugars are the main component of human milk. Though the disaccharide lactose is predominant amongst these sugars, human milk also contains over 150 sugars having more complex structure known as human milk oligosaccharides (HMOs). While lactose serves as an energy source for infants, HMOs are generally indigestible but provide a variety of other physiological benefits. For example, various HMOs promote development of beneficial intestinal microorganisms (the "prebiotic" effect), block adhesion of pathogens to gut epithelial surfaces and modulate the innate immune system; and are thought to play a role in brain development and neuronal activity.

The most abundant HMO is 2'-fucosyllactose (2'-FL), which consists of lactose fucosylated at the 2'-position of the galactose unit. 3-Fucosyllactose (3-FL), wherein lactose is fucosylated at the 3-position of the glucose unit, is also present in human milk. Most human milk oligosaccharides are fucosylated, unlike oligosaccharides produced by dairy animals.

Techniques have been developed for industrial scale production of HMOs, primarily for inclusion in infant formula products. For example, some HMOs (*e.g.,* 2'-FL and 3-FL) are synthesised by cultured microorganisms, such as recombinant *E. coli,* and then isolated from the broth of biomolecules produced by the culture through a series of purification steps. The final stages of purification of 2'-FL or 3-FL typically include one or more steps directed to drying the oligosaccharide in solution to remove as much solvent as possible so as to produce a powder that is convenient to store and transport. Various drying techniques have been reported in the art for sugars, including, for example, spray drying; fluid bed drying; flash drying; drying in a drum, cone, or spherical dryer; drying with an internal mill; vacuum drying; and combinations of two or more of the forgoing.

In spray drying in particular, the solution is dispersed into droplets and exposed to hot gas (typically air in the case of 2'-FL and 3-FL) inside a chamber of a spray dryer. The large surface area of the droplets causes the solvent to evaporate rapidly leaving solid powder particles. The wet air is vented from the chamber and the powder collected.

A dense powder is typically more efficient to store and transport than a less dense powder. Density of a powder is typically expressed as "loose bulk density", which is the weight of powder that fits into a given volume when poured into that volume, and "tapped bulk density", which is the weight of powder that fits into a given volume after it has been compacted into the volume by a defined number of standardised "taps".

There is a need for FL powders with high bulk density, and, accordingly, a process for obtaining the same, to increase efficiency of storage, transport and handling of the powder, thereby reducing costs to the producer, retailer, and, ultimately, consumer.

### SUMMARY

This specification generally provides processes for preparing a fucosyllactose (FL) by spray drying, as well as FL powder with high bulk density.

This specification provides, in part, a process for preparing dried FL, particularly dried 2'-FL or 3-FL. The process comprises:
providing an FL solution comprising 2'-FL and/or 3-FL in an aqueous medium,
feeding the FL solution into a spray dryer, and
spray drying the FL solution using the spray dryer.
Here, the FL solution comprises no organic solvent or less than 1% (by weight) of organic solvent. In some embodiments, the FL solution has a Brix value of from about 8 to about 75 %Brix before spray drying. In some embodiments, the FL solution is at a temperature of from about 2 to about 70°C before spray drying. In some embodiments, the spray dryer is a co-current spray dryer. In some embodiments, the spray dryer is a counter-current spray dryer. In some embodiments, the spray dryer is attached to an external fluid bed. In some embodiments, the spray dryer comprises an atomizer wheel, a rotary disk, a high pressure nozzle or a two fluid nozzle. In some embodiments, the spray dryer comprises an atomizer wheel. In some embodiments, the atomizer wheel operates at a speed of from about 10,000 to about 28,000 rpm. In some embodiments, the spray drying uses air at an inlet temperature of from 120 to 280°C. In some embodiments, the spray dryer has an air outlet temperature of from about 80 to about 110°C.

This specification also provides, in part, dried FL (particularly dried 2'-FL or 3-FL) prepared in accordance with this specification.

This specification also provides, in part, an FL in powder form, wherein the FL is 2'-FL or 3-FL. In some embodiments, the 2'-FL or 3-FL powder exhibits:
a loose bulk density of from about 500 to about 700 g/L,
a 100x tapped bulk density of from about 600 to about 850 g/L,
a 625x tapped bulk density of from about 600 to about 900 g/L and/or
a 1250x tapped bulk density of from about 650 to about 900 g/L.
In some embodiments, the FL powder has a moisture content of less than about 9.0% (by weight) water. In some embodiments, the FL powder has a moisture content of from about 3.0 to about 5.0% (by weight) water. In some embodiments, the FL powder has a moisture content of less than about 2.3% (by weight) water.

This specification also provides, in part, an FL (particularly 2'-FL or 3-FL) prepared in accordance with this specification for use in prevention and/or treatment of a disease.

This specification provides, in part, a prebiotic product comprising an FL (particularly 2'-FL or 3-FL) prepared in accordance with this specification.

This specification also provides, in part, a process for preparing a prebiotic product. The process comprises:
preparing dried FL (particularly dried 2'-FL or 3-FL) in accordance with this specification, and
admixing the dried FL with one or more prebiotic ingredients.

This specification also provides, in part, a process for preparing a prebiotic product. The process comprises admixing an FL powder described in this specification with one or more prebiotic ingredients.

This specification also provides, in part, a probiotic product comprising an FL (particularly 2'-FL or 3-FL) prepared in accordance with this specification.

This specification also provides, in part, a process for preparing a probiotic product. The process comprises:
preparing dried FL (particularly dried 2'-FL or 3-FL) in accordance with this specification, and
admixing the dried FL with one or more probiotic ingredients.

This specification also provides, in part, a process for preparing a probiotic product. The process comprises admixing an FL powder described in this specification with one or more probiotic ingredients.

This specification also provides, in part, a process for preparing a food. The process comprises:
preparing dried FL (particularly dried 2'-FL or 3-FL powder) in accordance with this specification, and
admixing the dried FL with one or more food ingredients.

This specification also provides, in part, a process for preparing a food. The process comprises admixing an FL powder described in this specification with one or more food ingredients.

This specification also provides, in part, a food comprising an FL (particularly 2'-FL or 3-FL) prepared in accordance with this specification.

In some of the above-described embodiments, the food comprises an infant formula.

Further benefits of the teachings of this specification will be apparent to one skilled in the art from reading this specification.

### BRIEF DESCRIPTION OF THE FIGURES

**Figure 1** shows 2'-FL powder prepared by the spray drying process described in **Example 1.**
**Figure 2** shows the solution that resulted from dissolving 28 grams of the 2'-FL powder prepared by the spray drying process described in **Example 1** in 180 mL of water at 40°C.
**Figure 3** shows the solution that resulted from dissolving the 3-FL powder prepared by the spray drying process described in **Example 7** in water.

### DETAILED DESCRIPTION

This detailed description is intended to acquaint others skilled in the art with Applicant's invention, its principles, and its practical application so that others skilled in the art may adapt and apply Applicant's invention in its numerous forms, as they may be best suited to the requirements of a particular use. This detailed description and its specific examples, while indicating certain embodiments, are intended for purposes of illustration only. This specification, therefore, is not limited to the described embodiments, and may be variously modified.

This specification provides a process for preparing a dried fucosyllactose (FL). In general, the process comprises feeding an FL solution comprising 2'-FL and/or 3-FL in an aqueous medium into a spray dryer, and spray drying the FL solution using the spray dryer.

### Fucosyllactose

The chemical structure of fucosyllactose (FL) consists of fucosylated lactose, *i.e.,* a lactose disaccharide linked to a fucose monosaccharide unit. Lactose consists of a α- or β-glucose sub-unit and a β-galactose sub-unit linked by a β1-4 glycosidic bond. The structures of lactose and fucose are depicted below.

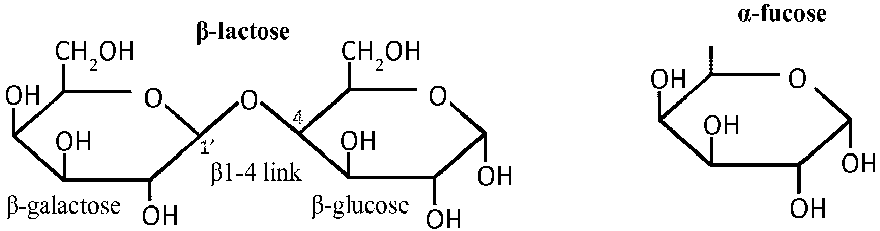

In 2'-FL, fucose is linked to 2'-carbon of galactose. In 3-FL, fucose is linked to 3-carbon of glucose. The structures of 2'-FL and 3-FL are depicted below.

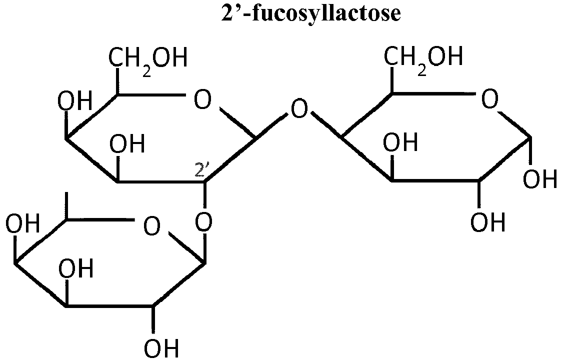

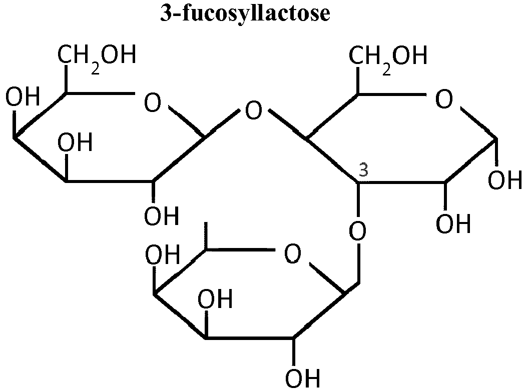

At room temperature and pressure, 2'-FL and 3-FL are typically a white to ivory coloured solid that is soluble in water.

### FL solution

An "FL solution" comprises an FL dissolved in an aqueous medium. An aqueous medium is a solvent comprising water. In some embodiments, the aqueous medium is pure water. In other embodiments, the medium comprises water with a trace amount of one or more organic solvents. In some such embodiments, the medium comprises less than 1% (by weight) organic solvent. In some embodiments, the medium comprises less than 0.1% (by weight) organic solvent. In some embodiments, the medium comprises less than 0.01% (by weight) organic solvent. In some embodiments, the medium comprises less than 0.001% (by weight) organic solvent. In some embodiments, the medium comprises less than 0.0001% (by weight) organic solvent.

In some embodiments, the FL solution is made before spray drying by dissolving in pure water FL crystals obtained from an earlier purification process. In some such embodiments, the FL solution comprises no organic solvent. In other embodiments, the FL solution comprises a trace amount of one or more organic solvents. In some such embodiments, the FL solution comprises less than 1% (by weight) organic solvent. In some embodiments, the FL solution comprises less than 0.1% (by weight) organic solvent. In some embodiments, the FL solution comprises less than 0.01% (by weight) organic solvent. In some embodiments, the FL solution comprises less than 0.001% (by weight) organic solvent. In some embodiments, the FL solution comprises less than 0.0001% (by weight) organic solvent.

In some embodiments, the FL solution has a Brix value of from about 8 to about 75 %Brix before spray drying. In some embodiments, the FL solution has a Brix value of from about 30 to about 65 %Brix before spray drying. In some embodiments, the FL solution has a Brix value of from about 50 to about 60 %Brix before spray drying. In some embodiments, the FL solution has a Brix value of about 50 %Brix before spray drying.

A "Brix value" indicates the sugar content of an aqueous solution. A Brix value can be expressed as a percentage (%Brix) or as "degrees Brix" (°Brix). %Brix is used in this specification. Strictly, a Brix value is the percentage by weight of sucrose in a pure water solution, and so does not apply to solutions comprising other solutes and/or solvents. However, a Brix value is simple to measure, and, therefore, is commonly used in the art as an approximation of the sugar content of sugar solutions other than pure sucrose solutions.

Techniques for measuring a Brix value are well known in the art. Dissolution of sugar in an aqueous solution changes the refractive index of the solution. Accordingly, an appropriately calibrated refractometer can be used to measure a Brix value of a solution. Alternatively, the density of a solution may be measured and converted to a Brix value. A digital density meter can perform this measurement and conversion automatically, or a hydrometer or pycnometer may be used.

In some embodiments, the FL solution comprises 2'-FL. In some embodiments, the FL solution comprises no 3-FL. In some embodiments, the FL solution comprises no fucosyllactose other than 2'-FL.

In some embodiments, the FL solution comprises 3-FL. In some embodiments, the FL solution comprises no 2'-FL. In some embodiments, the FL solution comprises no fucosyllactose other than 3-FL.

In some embodiments, the FL solution comprises both 2'-FL and 3-FL.

In some embodiments, the FL solution is at a temperature of from about 2 to about 70°C before spray drying. In some embodiments, the FL solution is at a temperature of from about 30 to about 60°C before spray drying. In some embodiments, the FL solution is at a temperature of from about 2 to about 30°C before spray drying. In some embodiments, the FL solution is at a temperature of about 30°C before spray drying. In some embodiments, the FL solution is at a temperature of about 20°C before spray drying. In some embodiments, the FL solution is at a temperature of from about 2 to about 5°C before spray drying. In some embodiments, the FL solution is heated or cooled to the desired temperature. For example, in some embodiments, the FL solution is pasteurized at, for example, 60°C before being passed through a sterile filter (*e.g.,* 0.2 µm sterile filter) and then collected into one or more holding tanks. Depending on the retention time in the holding tank, the pasteurized FL solution may cool naturally (for example, to a temperature of from about 30 to about 60°C). Alternatively, the holding tank(s) may include a heating and/or cooling jacket to maintain or adjust the temperature of the FL solution at a desired temperature, and/or the FL solution may be heated or cooled using a separate heat exchanger to achieve a desired temperature.

In some embodiments, the FL solution is at a temperature of from about 2 to about 70°C at the time it is fed into the spray dryer (immediately before it is dispersed into droplets in the spray dryer). In some embodiments, the FL solution is at a temperature of from about 30 to about 60°C immediately before it is dispersed into droplets in the spray dryer. In some embodiments, the FL solution is at a temperature of from about 2 to about 30°C at the time it is fed into the spray dryer. In some embodiments, the FL solution is at a temperature of about 30°C immediately before it is dispersed into droplets in the spray dryer. In some embodiments, the FL solution is at a temperature of about 20°C immediately before it is dispersed into droplets in the spray dryer. In some embodiments, the FL solution is at a temperature of from about 2 to about 5°C immediately before it is dispersed into droplets in the spray dryer.

### Spray drying

Spray drying is an industrial process for drying a liquid containing dissolved or dispersed solids. The liquid is dispersed into droplets and exposed to a flow of hot gas. The large total surface area of the droplets means that the liquid evaporates very quickly, leaving dry solid particles that form a powder.

Spray drying is performed in a drying chamber of a spray dryer machine. Various commercially available spray dryers may be suitable, such as, for example, a GEA Niro Production Minor Spray dryer (vol. = 1.24 m³) (Soeborg, Denmark); GEA Niro P 6.3 Spray dryer (vol. = 2.4 m³) (Soeborg, Denmark); and an SPX Anhydro CSD Type 70 Spray Dryer (vol. = ∼43 m³) (Soeborg, Denmark).

The term "before spray drying" refers to any point of the process before the liquid is dispersed into droplets.

Hot gas, which is used to dry the liquid, is typically pumped through the drying chamber by means of a fan. In some embodiments, the gas used to dry an FL solution is air. In some such embodiments, before entering the drying chamber, the air is sterilised by stepwise filtering with a pre-filter and a high efficiency particulate air (HEPA) filter. In general, a faster fan rate offers more drying energy and results in a lower moisture content of the dried product. A faster fan rate also tends to be helpful for achieving a greater degree of particle separation when using, for example, a separation cyclone.

Before entering the drying chamber, the air is typically heated to a desired temperature by, for example, an air steam heater, electrical heating elements, hot oil, direct gas burner or a combination of two or more of the foregoing. The "air inlet temperature" is the temperature of the air when it enters the drying chamber. In some embodiments, the air inlet temperature is from 120 to 280°C. In some embodiments, the air inlet temperature is from 120 to 210°C. In some embodiments, the air inlet temperature is from about 130 to about 190°C. In some embodiments, the air inlet temperature is about 180°C. In some embodiments, the air inlet temperature is from about 135 to about 160°C.

After heating, the air is pumped to an effective air distributor commonly on top of the drying chamber, which distributes the air in the correct pattern in the drying chamber.

The air throughput rate generally depends on various factors and the desired characteristics of the final dried product. For example, when atomization is achieved with atomizer wheel, a greater air throughput typically increases the moisture content and bulk density of the final powder product, reduces the retention time in the dryer, reduces the heat load of the spray-dried particles, reduces off-coloring of the product and/or reduces production costs associated with the spray-drying. For two fluid nozzle atomization, a greater air throughput rate normally produces smaller droplets from the nozzle, resulting in a decreased particle size distribution, bulk density and moisture content.

Moisture in the air generally equates to less vapor uptake capability. Moisture in the air also can result in humidity and stickiness of the final dried product. In some embodiments, to compensate for air humidity, the air outlet temperature is increased. In some embodiments, the air is dehumidified before being fed into the drying chamber. In some embodiments, the air is dehumidified before being heated.

The liquid to be dried (*i.e.,* the FL solution) may be fed into the spray dryer using, for example, a pump. In some embodiments, before entering the drying chamber the liquid is sterilised by filtering through, for example, an in-line 0.2 µm cartridge filter element.

The feed rate of the solution generally depends on various factors and the desired characteristics of the final dried product. In general, a faster feed rate tends to increase the moisture content of the dried product and increases the product stickiness (particularly for hygroscopic products). Faster solution feed rates also may result in a greater atomizer droplet size because more liquid is being atomized. In some embodiments, when a faster solution feed rate is used, an increased air outlet temperature is also used.

The spray dryer comprises an atomization means (*i.e.,* a mechanism that breaks up the liquid into droplets). The liquid passes through the atomization means as it enters the drying chamber. In some embodiments, the spray dryer comprises an atomizer wheel, a rotary disk, a high pressure nozzle or a two fluid nozzle. The centrifugal force of an atomizer wheel or rotary disk spinning at high speed throws out the liquid in a spray as it enters the wheel or disk. A high pressure nozzle creates a spray by forcing the liquid through an orifice at high pressure. A two fluid nozzle creates a spray by contacting the liquid with a compressed gas.

In some embodiments, the spray dryer comprises an atomizer wheel. The desired rotational speed of the atomizer wheel generally depends on various other process parameters and the desired characteristics of the final dried product. For example, variation of the atomizer speed can typically be used as a way to control the moisture content, bulk density and particle size distribution in the final dried product. In general, slower atomizer speeds result in a product with a greater moisture content, greater bulk density and greater particle size distribution relative to a product produced using a faster speed. A faster atomizer speed typically creates finer droplets in the atomization cloud. Normally, the finer droplets dry faster than less fine droplets, and the resulting product has a smaller particle size distribution relative to a product produced with slower atomizer speed. The smaller particles can be more difficult to separate, ultimately translating into less product percent yield recovery, such as when a separation cyclone is used. Particles made with a faster atomizer speed will generally have more entrapped and void air both inside the particles and between the particles. A faster atomizer speed can often be used to allow for an increased feed rate and/or air throughput to the dryer while still producing a product with minimal moisture content. Faster atomizer speeds can be particularly useful with feeds having a high viscosity, solids content or Brix value. Slower atomizer speeds can be particularly useful with feed solutions having a low solids content or Brix value. Slower atomizer speeds can often be used to achieve instant properties of the dried product, such as solubility, full dispersibility and wettability.

For high pressure atomization, pressure typically can be varied to control the moisture content, bulk density and particle size distribution in the final dried product. In general, increasing pressure results in effects similar to those resulting from increasing atomizer wheel speed.

For two fluid nozzle atomization, nozzle size typically can be varied to control the moisture content, bulk density and particle size distribution in the final dried product. In general, decreasing nozzle size results in effects similar to those resulting from increasing atomizer wheel speed.

In some embodiments, the atomizer wheel operates at a rotational speed of from about 10,000 to about 28,000 rpm. In some embodiments, the atomizer wheel operates at a speed of from about 10,000 to about 15,000 rpm. In some embodiments, the atomizer wheel operates at a speed of about 10,000 rpm. In some embodiments, the atomizer wheel operates at a speed of about 12,300 rpm. In some embodiments, the atomizer wheel operates at a speed of about 14,000 rpm. In some embodiments, the atomizer wheel operates at a speed of from about 20,000 to about 26,000 rpm. In some embodiments, the atomizer wheel operates at a speed of about 25,500 rpm. In some embodiments, the atomizer has a peripheral speed of from about 52 to about 147 meters/sec. In some embodiments, the atomizer diameter is about 120 mm, the atomizer speed is about 25,500 rpm, and the peripheral speed is about 160 meters/sec.

In some embodiments, solubility of the FL product is improved by varying one or more process conditions. In some embodiments, for example, the spray-drying is conducted in a manner that minimizes the exposure of the desired FL product to elevated temperatures. In some embodiments, the retention time inside the dryer is minimized. In some embodiments, a high air throughput is used. In some embodiments, the FL feed solution is not heated before spray-drying or is heated using a minimal temperature (*e.g*., no greater than 60°C) and/or minimal heating time before the spray-drying. In some embodiments, a low atomizer speed is used to obtain a larger particle size distribution. In some embodiments, a combination of the foregoing is used.

In some embodiments, the spray dryer is a co-current spray dryer. In some embodiments, the spray dryer is a counter-current spray dryer. In a co-current spray dryer, the air is blown into the drying chamber in the same direction as the liquid. In contrast, in a counter-current spray dryer the air and liquid enter the drying chamber in opposite directions.

In some embodiments, the air and liquid both enter the drying chamber from the top. The atomized liquid contacts the hot air and evaporates. The dried powder and the moisture-containing air are discharged from the bottom of the drying chamber into a separation cyclone, where the dried powder is separated from the air. The air is vented from the spray dryer into the atmosphere. The dried powder is collected from the separation cyclone.

The "air outlet temperature" is the temperature of the air when it exits the spray dryer. In some embodiments, the air outlet temperature is from about 80 to about 110°C. In some embodiments, the air outlet temperature is from about 100 to about 110°C. In some embodiments, the air outlet temperature is about 104°C.

In general, a higher solids content or Brix value in the feed solution means that there is less liquid to evaporate during the spray-drying process. This, in turn, generally allows for a lower air outlet temperature to be used, and tends to result in an increased bulk density, particle size distribution, separation efficiency (in, for example, a separation cyclone), and greater overall production rate.

In some embodiments, the spray dryer is attached to an external fluid bed. The dried powder is collected from the separation cyclone on the fluid bed and undergoes a second round of drying. In some embodiments, when an agglomerated product is desired, the fluid bed provides a humid environment that causes small particles to clump together, thereby creating a dried powder with larger particle size. Such powders are less dusty and flow more readily, which makes them easier to handle. In some such embodiments, water is added by, for example, two fluid nozzles to create the agglomeration.

### FL powder

This specification provides an FL prepared by the process disclosed in this specification.

In some embodiments, the FL product comprises 2'-FL. In some embodiments, the FL product comprises no 3-FL. In some embodiments, the FL product comprises no fucosyllactose other than 2'-FL.

In some embodiments, the FL product comprises 3-FL. In some embodiments, the FL product comprises no 2'-FL. In some embodiments, the FL product comprises no fucosyllactose other than 3-FL.

In some embodiments, the FL product comprises both 2'-FL and 3-FL.

This specification also provides an FL in powder form, wherein the FL is 2'-FL or 3-FL, having a loose bulk density of from about 500 to about 700 g/L, a 100x tapped bulk density of from about 600 to about 850 g/L, a 625x tapped bulk density of from about 600 to about 900 g/L and/or a 1250x tapped bulk density of from about 650 to about 1000 g/L.

"Bulk density" is the weight of the particles of a particulate solid (such as a powder) in a given volume, and is expressed in grams per litre (g/L). The total volume that the particles of a particulate solid occupy depends on the size of the particles themselves and the volume of the spaces between the particles. Entrapped air between and inside the particles also can affect the bulk density. Thus a particulate solid consisting of large, porous particles with large inter-particulate spaces will have a lower bulk density than a particulate solid consisting of small, non-porous particles that compact closely together. Bulk density can be expressed in two forms: "loose bulk density" and "tapped bulk density".

Loose bulk density (also known in the art as "freely settled" or "poured" bulk density) is the weight of a particulate solid divided by its volume where the particulate solid has been allowed to settle into that volume of its own accord (e.g. a powder poured into a container).

Closer compaction of a particulate solid within a container may be achieved by tapping the container and allowing the particles to settle more closely together, thereby reducing volume while weight remains the same. Tapping therefore increases bulk density. Tapped bulk density (also known in the art as "tamped" bulk density) is the weight of a particulate solid divided by its volume where the particulate solid has been tapped and allowed to settle into the volume a precise number of times. The number of times the particulate solid has been tapped is typically when stating the tapped bulk density. For example, "100x tapped bulk density" refers to the bulk density of the particulate solid after it has been tapped 100 times.

Techniques for measuring bulk density are well known in the art. Loose bulk density may be measured using a measuring cylinder and weighing scales: the particulate solid is poured into the measuring cylinder and the weight and volume of the particulate solid; weight divided by volume gives the loose bulk density. Tapped bulk density can be measured using the same technique, with the addition of tapping the measuring cylinder a set number of times before measuring weight and volume. Automation of tapping ensures the number, timing and pressure of individual taps is accurate and consistent. Automatic tapping devices are readily available, an example being the Jolting Stampfvolumeter (STAV 203) from J. Englesmann AG.

In some embodiments, the FL has a loose bulk density of from about 600 to about 700 g/L. In some embodiments, the FL has a loose bulk density of about 693 g/L. In some embodiments, the FL has a loose bulk density of from about 500 to about 600 g/L. In some embodiments, the FL has a loose bulk density of about 582 g/L.

In some embodiments, the FL has a 100x tapped bulk density of from about 750 to about 850 g/L. In some embodiments, the FL has a 100x tapped bulk density of about 814 g/L. In some embodiments, the FL has 100x tapped bulk density of from about 600 to about 700 g/L. In some embodiments, the FL has a 100x tapped bulk density of about 699 g/L.

In some embodiments, the FL has a 625x tapped bulk density of from about 750 to about 900 g/L. In some embodiments, the FL has a 625x tapped bulk density of about 833 g/L. In some embodiments, the FL has a 625x tapped bulk density of from about 700 to about 800 g/L. In some embodiments, the FL has a 625x tapped bulk density of about 771 g/L.

In some embodiments, the FL has a 1250x tapped bulk density of from about 850 to about 900 g/L. In some embodiments, the FL has a 1250x tapped bulk density of about 854 g/L. In some embodiments, the FL has a 1250x tapped bulk density of from about 750 to about 800 g/L. In some embodiments, the FL has a 1250x tapped bulk density of about 782 g/L.

In some embodiments, the FL has a loose bulk density of from about 600 to about 700 g/L, a 100x tapped bulk density of from about 750 to about 850 g/L, a 625x tapped bulk density of from about 750 to about 900 g/L and/or a 1250x tapped bulk density of from about 850 to about 900 g/L. In some embodiments, the FL has a loose bulk density of about 693 g/L, a 100x tapped bulk density of about 814 g/L, a 625x tapped bulk density of about 833 g/L and/or a 1250x tapped bulk density of about 854 g/L. In some embodiments, the FL has a loose bulk density of from about 500 to about 600 g/L, a 100x tapped bulk density of from about 600 to about 700 g/L, a 625x tapped bulk density of from about 700 to about 800 g/L and/or a 1250x tapped bulk density of from about 750 to about 800 g/L. In some embodiments, the FL has a loose bulk density of about 582 g/L, a 100x tapped bulk density of about 699 g/L, a 625x tapped bulk density of about 771 g/L and/or a 1250x tapped bulk density of about 782 g/L.

In some embodiments, the FL has a moisture content of less than 9% (by weight) water. In some embodiments, the FL has a moisture content of no greater than 5% (by weight) water. In some embodiments, the spray dryer is operated to achieve a moisture content of from about 3.0 to 5.0% (by weight). In some embodiments, the FL has a moisture content of less than 5% (by weight) water. In some embodiments, the FL has a moisture content of less than about 2.3% (by weight) water. In some such embodiments, the FL has a moisture content of from about 1.80 to about 2.25% (by weight) water. In some embodiments, the FL has a moisture content of from about 2.12 to about 2.21% (by weight) water. In some embodiments, the FL has a moisture content of from about 2.0 about 2.1% (by weight) water.

Techniques for measuring moisture content of a material are well known in the art. Examples include Karl-Fischer titration, wherein the quantity of Karl-Fischer solution absorbed by a sample indicates the amount of water in the sample, and gravimetric methods, wherein a sample is dried and weight loss due to evaporation of solvent is measured at intervals.

In some embodiments, the FL solution comprises 2'-FL, has a Brix value of 47.48 %Brix and is at a temperature of about 30°C before spray drying, the spray drying uses air with an inlet temperature of 135°C and an outlet temperature of 104°C, and the spray dryer is a co-current spray dryer comprising an atomizer wheel that operates at 25,136 rpm. The FL prepared is 2'-FL in powder form, has a loose bulk density of 601 g/L, a 100x tapped bulk density of 772 g/L, a 625x tapped bulk density of 832 g/L and a moisture content of 2.12-2.21% (by weight) water.

In some embodiments, the FL solution comprises 2'-FL, has a Brix value of 50.3 %Brix and is at a temperature of less than 35°C before spray drying, the spray drying uses air with an inlet temperature of 180°C and an outlet temperature of 102°C, and the spray dryer is a co-current spray dryer comprising an atomizer wheel that operates at 12,300 rpm. The FL prepared is 2'-FL in powder form, has a loose bulk density of 693 g/L, a 100x tapped bulk density of 814 g/L, a 625x tapped bulk density of 833 g/L and a 1250x tapped bulk density of 854 g/L.

In some embodiments, the FL solution comprises 3-FL, has a Brix value of 53.9 %Brix and is at a temperature of less than 5 °C before spray drying, the spray drying uses air with an inlet temperature of 180°C and an outlet temperature of 104°C, and the spray dryer is a co-current spray dryer comprising an atomizer wheel that operates at 25,500 rpm. The FL prepared is 3-FL in powder form, has a loose bulk density of 507 g/L, a 100x tapped bulk density of 625 g/L, a 625x tapped bulk density of 686 g/L, a 1250x tapped bulk density of 707 g/L and a moisture content of 2.0-2.1% (by weight) water.

In some embodiments, the FL solution comprises 3-FL, has a Brix value of 51.9 %Brix and is at a temperature of about 20°C before spray drying, the spray drying uses air with an inlet temperature of 180°C and an outlet temperature of 103°C, and the spray dryer is a co-current spray dryer comprising an atomizer wheel that operates at 25,500 rpm. The FL prepared is 3-FL in powder form, has a loose bulk density of 582 g/L, a 100x tapped bulk density of 699 g/L, a 625x tapped bulk density of 771 g/L and a 1250x tapped bulk density of 782 g/L.

In some embodiments, the FL is prepared by the process disclosed in this specification.

This specification additionally provides an FL as disclosed in this specification for use in prevention and/or treatment of a disease.

### Products comprising the dried FL

This specification provides a prebiotic product comprising the FL disclosed in this specification. A "prebiotic" is a substance that promotes growth of microorganisms beneficial to the host, and, in particular, those in the gastrointestinal tract. In some embodiments, a prebiotic product comprises multiple prebiotics, including the FL disclosed in this specification, to promote growth of a wide variety of beneficial microorganisms.

This specification also provides a process for preparing a prebiotic product, comprising conducting the process disclosed in this specification; obtaining the dried FL; and admixing the dried FL with one or more prebiotic product ingredients. Suitable prebiotic product ingredients are known in the art. In some embodiments, the prebiotic product comprises other prebiotic molecules, such as other HMOs and various plant polysaccharides such as inulin, pectin, β-glucan and xylooligosaccharide. In some embodiments, the prebiotic product ingredients comprises other sugars, such as lactose, glucose and galactose; thickeners, such as gum arabic; and acidity regulators, such as trisodium citrate.

This specification further provides a probiotic product comprising the FL disclosed in this specification. A "probiotic" product is typically a dietary supplement containing live microorganisms that replace or add to gastrointestinal microflora, to the benefit of the recipient.

This specification provides a process for preparing a probiotic product, comprising conducting the process disclosed in this specification; obtaining the dried FL; and admixing the dried FL with one or more probiotic product ingredients. Suitable probiotic product ingredients are known in the art. At least one probiotic product ingredient is a live microorganism (which is the "probiotic" itself). In some embodiments, multiple probiotic product ingredients are different live microorganisms. Examples of such microorganisms include *Lactobacillus* species such as *L. acidophilus* and *L. bulgaricus, Bifidobacterium* species such as *B. animalis* and *B. longum,* and *Saccharomyces boulardii.* Other probiotic ingredients include water, skimmed milk, substrates for microorganism growth such as sucrose, dextrose and/or other HMOs, and flavourings.

This specification also provides a food comprising the FL disclosed in this specification. In some embodiments, the food is an infant formula. Infant formula is a manufactured food for feeding to infants as a complete or partial substitute for human breast milk. In some embodiments, infant formula is sold as a powder and prepared for bottle- or cup-feeding to an infant by mixing with water. The molecular, and therefore nutritional, composition of infant formula is typically designed to be roughly the same as human breast milk. Accordingly, the FL disclosed in this specification is included in infant formula to provide nutritional benefits similar to those provided by HMOs in human breast milk.

This specification further provides a process for preparing a food, comprising conducting the process disclosed in this specification; obtaining the dried FL; and admixing the dried FL with one or more food ingredients. In some embodiments, the food is an infant formula. Suitable food ingredients for admixing with the dried FL include infant formula ingredients. Infant formula ingredients are known in the art and include lactose, vegetable oils such as palm, rapeseed, coconut and/or sunflower oil, fish oil, emulsifier such as soya lecithin, whey, casein, other HMOs, vitamins such as vitamins A, B₆, B₁₂, C and D, and minerals such as potassium citrate, calcium citrate, magnesium chloride, sodium chloride, sodium citrate and calcium phosphate.

### EXAMPLES

The following examples are merely illustrative, and not limiting to this specification in any way.

### Example 1

A solution of 2'-FL dissolved in water was spray dried to obtain 2'-FL in powder form. The 2'-FL solution had the characteristics shown in **Table 1.**

**Table 1**

| **Parameter** | **Value** |
|---|---|
| Total amount | 826 kg |
| Brix value | 47.48 %Brix |
| Calculated % solids | 45.5% |
| pH | 8.15 |
| Conductivity | 2 µS/cm |
| Protein concentration | 2.69 mg/L |
| Temperature | ∼30°C |

An SPX Anhydro CSD Type 70 (vol. = ∼43 m³) co-current spray dryer equipped with an atomizer wheel was used to spray dry the 2'-FL solution. The 2'-FL solution was fed into the spray dryer at an initial rate of 79 kg/h, which was increased to 115 kg/h over the course of the first 3 hours of spray drying. The spray dryer settings were as shown in **Table 2.**

**Table 2**

| **Parameter** | **Value** |
|---|---|
| Atomizer speed | 25,136 rpm |
| Air inlet flow | 3,000 m³/h |
| Air inlet temperature | 135°C |
| Air outlet temperature | 104°C |

The drying process lasted for 457 minutes.

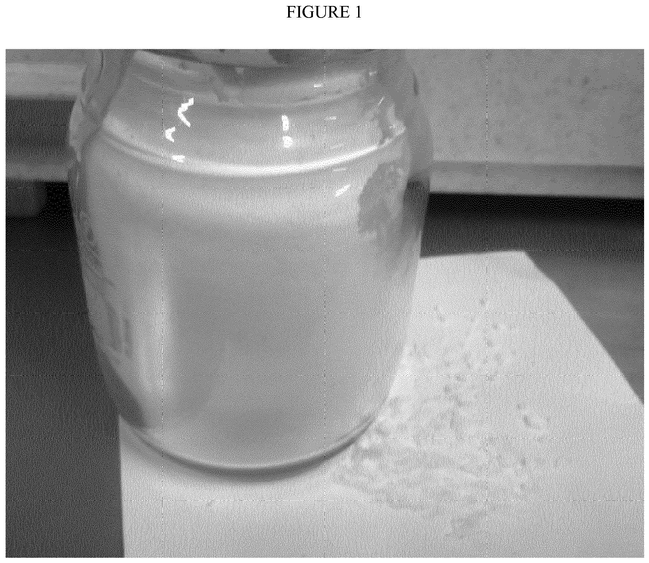

The dried 2'-FL powder obtained (shown in Figure 1) had the characteristics shown in **Table 3.**

**Table 3**

| **Parameter** | **Value** |
|---|---|
| Total amount | 340.1 kg |
| Product yield | 90.5% |
| Loose bulk density | 601 g/L |
| 100x tapped bulk density | 772 g/L |
| 625x tapped bulk density | 832 g/L |
| Moisture content (by weight) | 2.12-2.21% |

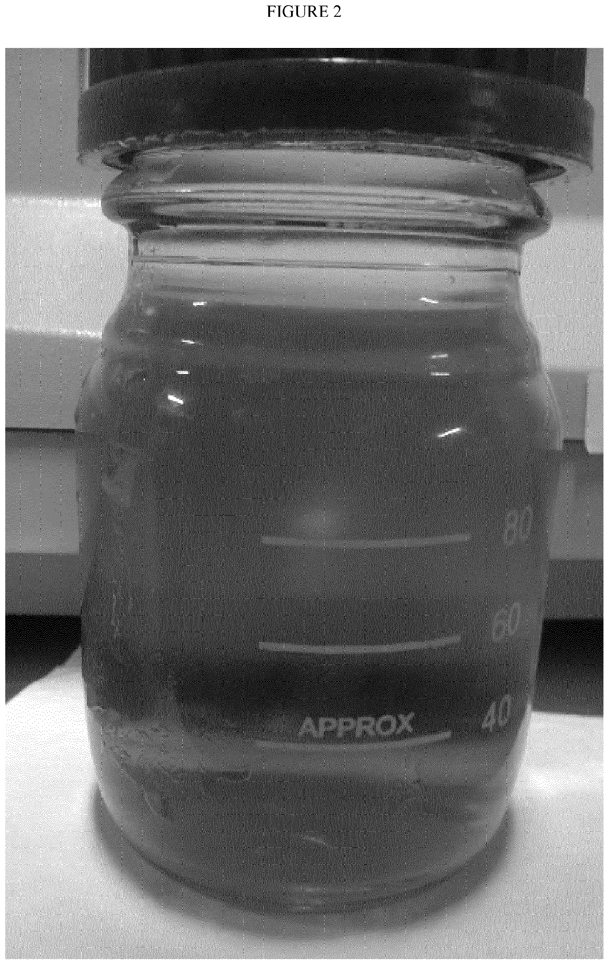

Bulk density of the 70 g of powder was measured using a Jolting Stampf Volumeter (STAV 203, J. Engelsmann AG), a 250 mL measuring cylinder and a technical weighing scale. Moisture content of the powder was measured using Karl-Fischer titration. 28 g of the 2'-FL powder were dissolved in 180 mL of water at 40 °C to prepare a colourless solution with no black spots or insoluble product (shown in Figure 2). The pH of the solution was 5.91 at 24°C.

### Examples 2-6

The process described in Example 1 was carried out a further five times. For each of these examples, the characteristics of the 2-FL solution and the dried 2'-FL product, as well as the spray dryer settings, are presented in **Table 4.**

**Table 4**

| **2'-FL Examples** | **2** | **3** | **4** | **5** | **6** |
|---|---|---|---|---|---|
| 2'-FL solution characteristics | | | | | |
| Temperature (°C) | ∼20 | <35 | ∼20 | ∼20 | ∼20 |
| Brix value (%) | 26.5 | 58-63 | 50.3 | 50.3 | 50.1 |

| Spray dryer settings | | | | | |
|---|---|---|---|---|---|
| Atomizer speed (rpm) | 12,300 | 12,300 | 10,000 | 14,000 | 25,500 |
| Air inlet temperature (°C) | 180 | 180 | 183 | 181 | 179 |
| Air outlet temperature (°C) | 102 | 102 | 103 | 102 | 103 |

| 2'-FL dried product characteristics | | | | | |
|---|---|---|---|---|---|
| Loose bulk density (g/L) | 583 | 693 | 682 | 648 | 642 |
| 100x tapped bulk density (g/L) | unknown | 814 | 765 | 753 | 686 |
| 625x tapped bulk density (g/L) | 686 | 833 | 789 | 778 | 761 |
| 1250x tapped bulk density (g/L) | 686 | 854 | 798 | 787 | 761 |

### Example 7

A solution of 3-FL dissolved in water was spray dried to obtain 3-FL in powder form. The 3-FL solution had the characteristics shown in **Table 5.**

**Table 5**

| **Parameter** | **Value** |
|---|---|
| Total amount | 46 kg |
| Brix value | 53.9%Brix |
| Temperature | <5°C |

A GEA Niro Production Minor (vol. = 1.24 m³) co-current spray dryer equipped with an atomizer wheel was used to spray dry the 3-FL solution. The 3-FL solution was fed into the spray dryer at a rate of 5-15 kg/h, and more specifically, approximately 6 kg/h. The spray dryer settings were as shown in **Table 6.**

**Table 6**

| **Parameter** | **Value** |
|---|---|
| Atomizer speed | 25,500 rpm |
| Air inlet flow | 360 kg/h |
| Air inlet temperature | 180°C |
| Air outlet temperature | 104°C |

The drying process lasted for 532 minutes.

The dried 3-FL powder obtained had the characteristics shown in **Table 7.**

**Table 7**

| **Parameter** | **Value** |
|---|---|
| Total amount | 23 kg |
| Product yield | 89.3% |
| Loose bulk density | 507 g/L |
| 100x tapped bulk density | 625 g/L |
| 625x tapped bulk density | 686 g/L |
| 1250x tapped bulk density | 707 g/L |
| Moisture content (by weight) | 2.0-2.1% |

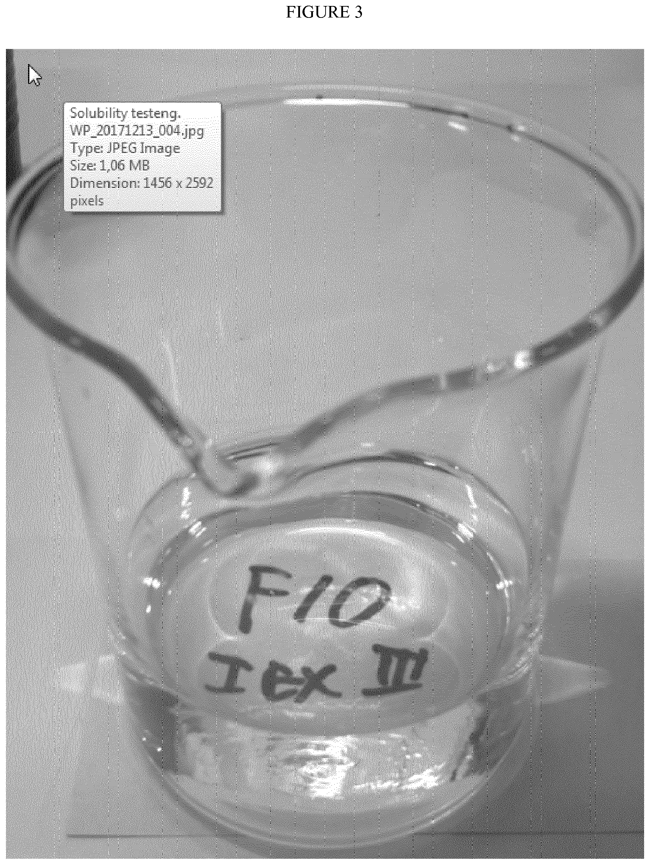

Bulk density of the 70 g of powder was measured using a Jolting Stampf Volumeter (STAV 203, J. Engelsmann AG), a 250 mL measuring cylinder and a technical weighing scale. Moisture content of the powder was measured using Karl-Fischer titration. A sample of the 3-FL powder was dissolved in water, producing a colourless solution with no black spots or insoluble product (shown in Figure 3).

### Examples 8-10

The process described in Example 7 was carried out a further three times. For each of these examples, the characteristics of the 3-FL solution and the dried 3-FL product, as well as the spray dryer settings, are presented in the **Table 8.**

**Table 8**

| **3-FL Examples** | **8** | **9** | **10** |
|---|---|---|---|
| 3-FL solution characteristics | | | |
| Temperature (°C) | ∼20 | <5 | <5 |
| Brix value (%) | 51.9 | 58.3 | 31.2 |

| Spray dryer settings | | | |
|---|---|---|---|
| Atomizer speed (rpm) | 25,500 | 25,500 | 25,500 |
| Air inlet temperature (°C) | 180 | 180 | 180 |
| Air outlet temperature (°C) | 103 | 104 | 104 |

| 3-FL dried product characteristics | | | |
|---|---|---|---|
| Loose bulk density (g/L) | 582 | 534 | 556 |
| 100x tapped bulk density (g/L) | 699 | 631 | 648 |
| 625x tapped bulk density (g/L) | 771 | 737 | 753 |
| 1250x tapped bulk density (g/L) | 782 | 761 | 753 |

The words "comprise", "comprises" and "comprising" are to be interpreted inclusively rather than exclusively. This interpretation is intended to be the same as the interpretation that these words are given under United States patent law at the time of this filing.

The singular forms "a" and "an" are intended to include plural referents unless the context dictates otherwise. Thus, for example, a reference to the presence of "an excipient" does not exclude the presence of multiple excipients unless the context dictates otherwise.

## Claims

1. A process for preparing a dried fucosyllactose, wherein:
the process comprises:
providing a fucosyllactose solution comprising 2'-fucosyllactose and/or 3-fucosyllactose in an aqueous medium,
feeding the fucosyllactose solution into a spray dryer, and
spray drying the fucosyllactose solution using the spray dryer; and
the fucosyllactose solution comprises no organic solvent or less than 1% (by weight) of organic solvent.

2. A process according to claim 1, wherein the fucosyllactose solution comprises 2'-fucosyllactose.

3. A process according to claim 1 or 2, wherein the fucosyllactose solution comprises 3-fucosyllactose.

4. A process according to any of the preceding claims, wherein the fucosyllactose solution comprises less than 0.001% (by weight) organic solvent.

5. A process according to any of the preceding claims, wherein the fucosyllactose solution comprises no organic solvent.

6. A process according to any one of the preceding claims, wherein the fucosyllactose solution has a Brix value of from 30 to 65 %Brix before spray drying.

7. A process according to any one of the preceding claims, wherein the fucosyllactose solution is at a temperature of from 30 to 60°C immediately before being dispersed into droplets in the spray dryer.

8. A process according to any one of claims 1-6, wherein the fucosyllactose solution is at a temperature of from 2 to 30°C immediately before being dispersed into droplets in the spray dryer.

9. A process according to any one of the preceding claims, wherein:
the spray dryer comprises an atomizer wheel, and
the atomizer wheel is operated at a rotational speed of from 10,000 to 28,000 rpm.

10. A process according to any one of the preceding claims, wherein the spray drying uses air having an air inlet temperature of from 130 to 190°C.

11. A process according to any one of the preceding claims, wherein the spray drying uses air having an air outlet temperature of from 80 to 110°C.

12. A fucosyllactose powder, wherein:
the fucosyllactose powder comprises spray-dried 2'-fucosyllactose or 3-fucosyllactose, and
the fucosyllactose powder exhibits:
a loose bulk density of from 500 to 700 g/L,
a 100x tapped bulk density of from 600 to 850 g/L,
a 625x tapped bulk density of from 600 to 900 g/L, and/or
a 1250x tapped bulk density of from 650 to 900 g/L.

13. A fucosyllactose powder according to claim 12, wherein the fucosyllactose powder has a moisture content of less than 9% (by weight) water.

14. A fucosyllactose powder according to claim 12 or 13, wherein the fucosyllactose powder comprises 2'-fucosyllactose.

15. A fucosyllactose powder according to any one of claims 12-14, wherein the fucosyllactose powder comprises 3-fucosyllactose.
